(19) Europäisches Patentamt
European Patent Office
Office européen des brevets

(11) EP 2 681 224 B1

(12) **EUROPEAN PATENT SPECIFICATION**

(45) Date of publication and mention of the grant of the patent:
**22.06.2016 Bulletin 2016/25**

(21) Application number: **12706836.9**

(22) Date of filing: **01.03.2012**

(51) Int Cl.:
***C07D 501/12** (2006.01)*

(86) International application number:
**PCT/EP2012/053483**

(87) International publication number:
**WO 2012/117038 (07.09.2012 Gazette 2012/36)**

# (54) DEGRADATION OF PENICILLIN COMPOUNDS

ABBAU VON PENICILLINVERBINDUNGEN

DÉGRADATION DE COMPOSÉS DE PÉNICILLINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.03.2011 EP 11156792**

(43) Date of publication of application:
**08.01.2014 Bulletin 2014/02**

(73) Proprietor: **DSM Sinochem Pharmaceuticals Netherlands B.V.**
**2613 AX Delft (NL)**

(72) Inventor: **DEKKERS, Rocus, Marinus**
**NL-6100 AC Echt (NL)**

(74) Representative: **De Vroom, Erik**
**DSM Intellectual Property**
**P.O. Box 4**
**6100 AA Echt (NL)**

(56) References cited:
**WO-A1-98/48036 GB-A- 1 259 204**

- **Chain: "Chapter 21, Physical and Chemical Properties of the Penicillins" In: Florey et al. (ed.): "Antibiotics, Volume II", 1949, Oxford University Press, London, XP002635018, page 802, cited in the application Section "Sodium bisulphite".**
- **Wintersteiner et al.: "Chapter VIII: Some inactivation and degradation reactions not included in Chapters IV and VII" In: Clarke et al. (eds.): "The Chemistry of Penicillin", 1949, Princeton University Press, Princeton, XP009147356, pages 207-242, cited in the application Pages 221-222: "The Inactivation of Benzylpenicillin by Sodium Sulfite".**
- **TERAO ET AL.: "Studies on the formulation and admixture of parenteral preparations. II. Degradation of penicillins and cephalosporins by sodium bisulfite", YAKUGAKU ZASSHI (J. PHARM. SOC. JAPAN), vol. 102, no. 10, 1982, pages 978-983, XP009147358, ISSN: 0031-6903**
- **DESHPANDE ET AL.: "Degradation of beta-lactam antibiotics", CURRENT SCIENCE, vol. 87, no. 12, 25 December 2004 (2004-12-25), pages 1684-1695, XP002635019, Bangalore (INDIA)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 681 224 B1

## Description

### Field of the invention

The present invention relates to the degradation of β-lactam compounds such as penicillins in the presence of other β-lactam compounds such as cephalosporins. Furthermore the present invention relates to the use of sulfite in the degradation of penicillins, for example in industrial waste streams.

### Background of the invention

β-Lactam antibiotics are advantageously used already for several decades in order to cure mankind from all kinds of infectious diseases. Nevertheless, under certain circumstance, β-lactams can be harmful and there are strict requirements on the absence of β-lactams in certain products, such as food products, waste, but also certain drugs and even antibiotic products themselves. For instance, β-lactam antibiotics of the cephalosporin type preferably do not contain β-lactam antibiotics of the penicillin type and vice versa. The Pharmacopeia contains requirements of the purity of cephalosporins with respect to penicillins.

7-Aminodesacetoxycephalosporanic acid (7-ADCA) is the starting material for cephalosporin type β-lactam antibiotics such as cephalexin, cefadroxil and cephradine. The traditional route to 7-ADCA starts with penicillin G and thereby has an inherent risk that residual penicillin compounds such as penicillin G and 6-aminopenicillanic acid (6-APA) will be present in 7-ADCA. Novel production of 7-ADCA via fermentation techniques using a *Penicillium* strain transformed with an expandase gene as disclosed in WO 93/05158, WO 95/04148, WO 95/04149, WO 98/48036, WO 98/48034, and/or WO 98/48035 inherently have, at various stages in the process, contaminating levels of penicillin-compounds such as 6-APA. There is therefore a need for methods to selectively degrade β-lactam compounds, for instance to selectively degrade 6-APA.

### Detailed description of the invention

In general, β-lactams are unstable compounds and there exist several methods to degrade β-lactams. However, the existing methods use either harsh reaction conditions and/or lack selectivity and/or are expensive and/or laborious. For instance, 6-APA may be degraded selectively by incubation at selected temperature- and pH-ranges or in the presence of $CO_2$ and other sources of $CO_2$ or by the non-catalytic reaction with a mercaptoamine as described in GB 1259204. Also, β-lactamases can be used for selective degradation. The inactivation of $\Delta^2$-pentenylpenicillin and penicillin G using sodium bisulfite has been reported by Florey et al. ("Antibiotics", Volume II (1949) Oxford University Press, London, 802) and by Clarke et al. ("The Chemistry of Penicillin" (1949) Princeton University Press, Princeton, 207-242) although these studies do not suggest any selectivity related with sodium bisulfite. Finally, several purification methods are known that separate 6-APA or other penicillins from cephalosporin products.

In a first aspect, it has surprisingly been found that a selective degradation of penicillin compounds can be obtained by carrying out the degradation in the presence of sulfite. Thus, the invention provides a process for the degradation of a penicillin compound of general formula (1) in a mixture comprising said compound of general formula (1) and a cephalosporin compound of general formula (2).

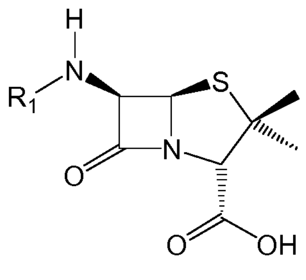

(**1**)

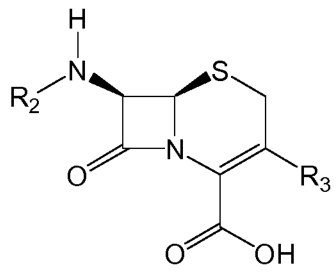

(**2**)

In the compounds of general formula (1) and (2) $R_1$ and $R_2$ are hydrogen or acyl groups such as, for example, adipyl, 2-aminoadipyl, 2-amino-4-thiazolylacetyl, 2-amino-4-thiazolyl(methoxyimino)acetyl, 2,5-dihydrophenylglycyl, 2-furanyl(methoxy-imino)acetyl, glutaryl, hydroxyphenylacetyl, 4-hydroxyphenylglycyl, phenoxyacetyl, phenylacetyl, phenylglycyl, 4-pyridinylthioacetyl, 1H-tetrazol-1-ylacetyl or 2-thienylacetyl. The aforementioned groups are widely present in the majority of β-lactams with therapeutic use. For similar purposes, the group $R_3$ in the compound of general formula

(2) can be hydrogen or a moiety such as acetamidomethyl, acetoxymethyl, chlorine, ethenyl, hydrogen, hydroxymethyl, methoxy, methoxymethyl, methyl, methylpyridinium, (1-methyl-1H-tetrazol-5-ylthio)methyl, propenyl, 1,2,5,6-tetrahydro-2-methyl-5,6-dioxo-1,2,4-triazin-3-ylthio)methyl or (1,3,4-thiadiazol-2-ylthio)methyl. Examples of compounds of general formula (2) from which penicillin compounds may be removed according to the present invention are 7-aminocephalosporanic acid (7-ACA), 7-ADCA, adipyl-7-ADCA, cefaclor, cefadroxil, cefamandole, cefatrizine, cefazedone, cefazolin, cefdinir, cefixime, cefmenoxime, cefodizime, cefonicid, cefoperazone, ceforanide, cefotaxime, cefotiam, cefpimizole, cefpiramide, cefprozil, cefpodoxime, cefroxadine, cefsulodin, ceftazidime, cefteram, ceftezole, ceftibuten, ceftizoxime, ceftriaxone, cefuroxime, cefuzonam, cephalexin, cephaloglycin, cephaloridine, cephalosporin C, cephalothin, cephapirin and cephradine.

The penicillin compound of general formula (1) preferably is selected from the group consisting of 6-APA and any 6-N-acyl derivative such adipyl-6-APA, penicillin G, penicillin V, amoxicillin, ampicillin or those disclosed in the prior art cited above.

In a preferred embodiment, the penicillin compound of general formula (1) is 6-APA or adipyl-6-APA. In another preferred embodiment the cephalosporin compound of general formula (2) is 7-ADCA or any 7-N-acyl derivative of 7-ADCA such as adipyl-7-ADCA. Most preferred is degradation of 6-APA and/or adipyl-6-APA in a mixture comprising 7-ADCA and/or adipyl-7-ADCA.

The process may be carried out at any suitable temperature, such as a temperature between 0 and 60°C, preferably between 10 and 40°C. The skilled person is very well capable of selecting the appropriate temperature for the degradation of the penicillin compound in the process of the invention.

In the context of the present invention the term "sulfite" (or "sulphite") refers to any compound comprising sulfur in oxidation state (also referred to as oxidation number or valency) $4^+$. Preferred examples are $SO_2$, $(HSO_3)^-$ and $(SO_3)^{2-}$ which exist next to one another in aqueous solutions in ratio's depending on the pH. For example, at pH 1 an aqueous solution contains approximately 90% $SO_2$ and approximately 10% $(HSO_3)^-$, at pH 7 an aqueous solution contains approximately 50% $(HSO_3)^-$ and approximately 50% $(SO_3)^{2-}$ and at pH 8 an aqueous solution contains approximately 10% $(HSO_3)^-$ and approximately 90% $(SO_3)^{2-}$. Sulfite may be added in any form. A suitable source is $NaHSO_3$ but other sources such as metal salts of $(HSO_3)^-$ or $(SO_3)^{2-}$ or $(S_2O_3)^{2-}$ (meta bisulfite) may be used as well. Also, gaseous $SO_2$ may be brought into the reaction mixture. The skilled person is very well capable of selecting the appropriate source of sulfite that can be used in the process of the invention.

Preferably the process is carried out at a pH value between 3 and 9, more preferably between 4 and 8, more preferably between 5 and 7, most preferably between 5.5 and 6.5. Preferably the pH is such that at least 10% of sulfite is present as $(HSO_3)^-$ which usually is between pH 1 and 8; more preferably the pH is such that at least 40% of sulfite is present as $(HSO_3)^-$ which usually is between pH 1.6 and 7.2, most preferably the pH is such that at least 80% of sulfite is present as $(HSO_3)^-$ which usually is between pH 2.5 and 6.3.

In the context of the present invention, "degradation of a penicillin compound" is defined as that in the penicillin compound preferentially the 4-membered β-lactam-ring is cleaved, resulting in a degradation product of the penicillin compound. Preferably, the rate constant $k_{(1)}$ (in $hr^{-1}.(g/l)^{-1}$) representing degradation of the compound of general formula (1) is 10 times the rate constant $k_{(2)}$ (in $hr^{-1}.(g/l)^{-1}$) representing degradation of the compound of general formula (2). More preferably $k_{(1)}$ is 10 to 100 times the rate constant $k_{(2)}$ and most preferably $k_{(1)}$ is 50 to 1000 times the rate constant $k_{(2)}$.

In another aspect, the invention provides the use of sulfite for the degradation of a penicillin compound, more preferably a penicillin compound selected from the group consisting of 6-APA and any 6-N-acyl derivative such adipyl-6-APA, penicillin G, penicillin V, amoxicillin, ampicillin or those disclosed in the prior art cited above. In one embodiment, the invention provides the use of sulfite for the degradation of a penicillin compound wherein the penicillin compound is selectively degraded in the presence of a cephalosporin compound. A preferred embodiment is the use of sulfite for the degradation of a contaminating penicillin compound in a process for the production of a cephalosporin compound. In another embodiment, the invention provides the use of sulfite for the degradation of penicillin in a waste stream, such as a mycelium of a production organism used for the production of β-lactam antibiotic compounds such as penicillin producing microorganisms from the genus *Penicillium.* Said use has significant practical importance as traces of penicillins in waste streams are hazardous for the public health in view of unwanted and/or uncontrolled development of bacterial resistance against penicillins.

## EXAMPLES

### Materials and methods

The concentration of 6-APA was determined by HPLC using a Dionex Ultimate 3000 RS equipped with a Lichrospher RP-18 column (5 μm, 250*4.6 mm (Merck)) operated at a flow of 1.3 ml/min. Other conditions:

- Eluens (isocratic): 6.62 g $K_2HPO_4$ + 17.76 g $KH_2PO_4$ are dissolved in purified water. 100 ml acetonitrile (HPLC grade) is added. The volume is adjusted to a total of 2 liter by addition of purified water
- Run time 12 min
- Injection volume 10 $\mu$l
- Wavelength for detection: 214 nm
- Temperature: 20-25 °C

Under these conditions 6-APA eluted at 2.9 minutes and pHPG at 1.5 min.

Sulfite was determined using the Reflectoquant sulfite test (Merck prod. nr. 1.16987.0001) measured on an RQflex 2 (Merck prod. nr. 1.16970.0001).

## Example 1

### Degradation of 6-APA by $NaHSO_3$

The degradation of 6-APA ((1), $R_1$ = H) was measured as a function of time, bisulfite concentration (0-8 g/l) and at several pH-values (4, 6 and 9). Solutions containing 1 g/l 6-APA in water were brought to the desired pH using 1 N NaOH and/or 1 N $H_2SO_4$. Then the indicated amounts of $NaHSO_3$ were added. The various mixtures were incubated at 20°C and samples were taken in time in order to measure the residual 6-APA concentration as well as remaining sulfite concentration. Sulfite can be present in the solution as $H_2SO_3$, $HSO_3^-$, or $SO_3^{2-}$, depending on the pH. $H_2SO_3$ has two pKa values, one at -1.8 and a second at -7.0. At pH values 4 and 6, virtually all sulfite is present as $HSO_3$ while at pH 9 virtually all sulfite is present as $SO_3^{2-}$. Tables 1a-1c show the time-dependent degradation of 6-APA by sulfite at pH 4, 6 and 9 respectively.

Table 1 a: 6-APA degradation as a function of the sulfite concentration at pH=4

| Time (h) | Sodium bisulfite (g/l) | | | |
|---|---|---|---|---|
| | 0 | 2 | 8 | |
| | 6-APA [g/l] | 6-APA [g/l] | 6-APA [g/l] | |
| 0.00 | 0.98 | 0.98 | 0.93 | |
| 1.00 | | 0.89 | 0.57 | |
| 1.75 | | | 0.48 | |
| 2.00 | | 0.83 | | |
| 3.00 | | 0.80 | | |
| 4.00 | | 0.74 | | |
| 4.75 | | | 0.18 | |
| 5.50 | | 0.65 | | |
| 22.00 | 0.81 | 0.18 | 0.00 | |
| | | | | |
| k' | 0.008 | 0.076 | 0.396 | Average |
| k | | 0.034 | 0.049 | 0.041 |

Table 1b: 6-APA degradation as a function of the sulfite concentration at pH=6

| Time (h) | Sodium bisulfite (g/l) | | | |
|---|---|---|---|---|
| | 0 | 2 | 8 | |
| | 6-APA [g/l] | 6-APA [g/l] | 6-APA [g/l] | |
| 0 | 1.02 | 1.01 | 0.98 | |
| 2 | | 0.73 | 0.15 | |

(continued)

| Time (h) | Sodium bisulfite (g/l) | | | |
|---|---|---|---|---|
| | 0 | 2 | 8 | |
| | 6-APA [g/l] | 6-APA [g/l] | 6-APA [g/l] | |
| 3.5 | | 0.58 | 0.03 | |
| 4.5 | | 0.50 | 0.01 | |
| 5.5 | | 0.45 | 0.00 | |
| 22 | 1.00 | 0.07 | 0.00 | |
| 23 | | 0.07 | | |
| 26 | | 0.05 | | |
| 29 | | 0.04 | | |
| | | | | |
| k' | 0.000 | 0.151 | 0.950 | Average |
| k | | 0.076 | 0.119 | 0.097 |

Table 1c: 6-APA degradation as a function of the sulfite concentration at pH=9

| Time (h) | Sodium bisulfite (g/l) | | | |
|---|---|---|---|---|
| | 0 | 2 | 8 | |
| | 6-APA [g/l] | 6-APA [g/l] | 6-APA [g/l] | |
| 0 | 1.01 | 1.00 | 0.96 | |
| 1 | | 0.99 | 0.67 | |
| 2 | | 1.01 | 0.68 | |
| 3 | | 1.00 | 0.68 | |
| 20 | 1.02 | 0.93 | 0.61 | |
| | | | | |
| k' | 0.000 | 0.003 | 0.034 | Average |
| k | | 0.0017 | 0.0042 | 0.0029 |

The results show that the degradation rate depends on both the 6-APA concentration [6-APA] and the (bi)sulfite-concentration [$SO_3^{2-}$] and is therefore at least second order. The reaction could be modeled using a second order rate equation:

$$r = k[6APA][SO_3^{2-}]$$

in which k is the rate coefficient or rate constant (in $hr^{-1}.(g/l)^{-1}$).

The $SO_3^{2-}$-concentrations measured during the reactions show that the concentration remains quite constant during the degradation (not shown). Sulfite was present in excess. Therefore the rate equation could be simplified to a pseudo first order rate equation:

$$r = k'[6APA]$$

in which $k' = k[SO_3^{2-}]$ (in $hr^{-1}$).

At pH 4, 6 and 9 for each sulfite-concentration k' and subsequently k were calculated (see Tables 1a-1c). The fastest degradation rate of 6-APA is found at pH 6. From the calculated k-values it can be deduced that the degradation at pH 9 is approximately 30-fold slower compared to pH 6. At pH 4, the degradation rate is 2.5-fold lower, however still significant.

## Example 2

### Degradation of adipyl-7-ADCA by $NaHSO_3$ at pH 7

The degradation of adipyl-7-ADCA **((2),** $R_2$ = adipyl, $R_3$ = $CH_3$) was measured as a function of time, bisulfite concentration (0-12 g/l) and at pH=7. For experimental details and analysis see Example 1.

Table 2: Adipyl-7-ADCA degradation as a function of sulfite concentration at pH=7

| Time (h) | Sodium bisulfite (g/l) | | | |
|---|---|---|---|---|
| | 0 | 6 | 12 | |
| | Adipyl-7-ADCA [g/l] | Adipyl-7-ADCA [g/l] | Adipyl-7-ADCA [g/l] | |
| 0 | 27.05 | 26.52 | 25.88 | |
| 15 | 27.08 | 26.36 | 25.49 | |
| 48 | 26.72 | 24.97 | 23.17 | |
| | | | | |
| k' | | 0.0013 | 0.0023 | Average |
| k | | 0.00022 | 0.0001 | 0.00020 |

The data in table 2 show that adipyl-7-ADCA is much more resistant against the degradation by sulfite. Hardly any degradation takes place. This is illustrated by comparing the second order rate constant for 6-APA at pH=6 (0.097) with the second order rate constant for adipyl-7-ADCA at pH=7 (0.00020) which shows that 6-APA is degraded 485 times faster than adipyl-7-ADCA.

## Example 3

### Degradation of 7-ADCA by different concentrations of $NaHSO_3$

The degradation of 7-ADCA **((2),** $R_2$ = H, $R_3$ = $CH_3$) was measured as a function of time at bisulfite concentrations of 0 g/l, 4 g/l, 13 g/l and 16 g/l. Four 7-ADCA solutions (50 ml) were stirred at 20°C for 2 days. Concentrations of 7-ADCA were measured by HPLC, accuracy ± 2%. The pH was adjusted to 7.6 with 1 M NaOH or to pH 9.4 as indicated in the below Table.

Table 3: 7-ADCA degradation as a function of the sulfite concentration

| Time (min) | Sodium bisulfite (g/l) 0 | | 13 | | 16 | | 4 | |
|---|---|---|---|---|---|---|---|---|
| | pH 7.6 | | pH 7.6 | | pH 7.6 | | pH 9.4 | |
| | 7-ADCA mM | % | 7-ADCA mM | % | 7-ADCA mM | % | 7-ADCA mM | % |
| 0 | 102.06 | 100.0 | 96.62 | 100.0 | 113.40 | 100.0 | 126.17 | 100.0 |
| 30 | | | 92.33 | 95.6 | | | | |
| 40 | | | | | 114.84 | 101.3 | | |
| 60 | 99.97 | 98.0 | | | | | 126.58 | 100.3 |
| 75 | | | 94.07 | 97.4 | | | | |
| 90 | | | | | 113.65 | 100.2 | | |

(continued)

| Time (min) | Sodium bisulfite (g/l) 0 | | 13 | | 16 | | 4 | |
|---|---|---|---|---|---|---|---|---|
| | pH 7.6 | | pH 7.6 | | pH 7.6 | | pH 9.4 | |
| | 7-ADCA | | 7-ADCA | | 7-ADCA | | 7-ADCA | |
| | mM | % | mM | % | mM | % | mM | % |
| 120 | | | | | | | 126.50 | 100.3 |
| 125 | | | 94.17 | 97.5 | | | | |
| 130 | 100.55 | 98.5 | | | | | | |
| 180 | | | 93.41 | 96.7 | 114.06 | 100.6 | | |
| 200 | | | | | | | 123.11 | 97.6 |
| 225 | 99.61 | 97.6 | | | | | | |
| 285 | | | | | 116.14 | 102.4 | | |
| 300 | | | | | | | 124.97 | 99.1 |
| 310 | | | 93.43 | 96.7 | | | | |
| 325 | 99.02 | 97.0 | | | | | | |
| 1740 | 100.85 | 98.8 | 92.92 | 96.2 | 112.54 | 99.2 | 123.73 | 98.1 |
| 2880 | 100.26 | 98.2 | 91.25 | 94.4 | 110.75 | 97.7 | 122.64 | 97.2 |
| 2880 (dupl.) | 101.85 | 99.8 | 92.14 | 95.4 | 114.02 | 100.5 | 125.11 | 99.2 |

**Example 4**

**Degradation of 7-ADCA by $NaHSO_3$ at pH 10**

The degradation of 7-ADCA **((2),** $R_2$ = H, $R_3$ = $CH_3$) was measured as a function of time at a bisulfite concentration of 3 g/l and at pH=10. For experimental details and analysis see Example 1.

Table 4: 7-ADCA degradation as a function of time at pH=10 and a bisulfite concentration of 3 g/l.

| Time | 7-ADCA |
|---|---|
| 0 | 45.91 |
| 1 | 45.88 |
| 19 | 44.9 |

**Example 5**

**Degradation of 6-APA by $NaHSO_3$ in the presence of 7-ADCA and adipyl-7-ADCA**

The pH of mixtures (500 ml) comprising 6-APA **((1),** $R_1$ = H), 7-ADCA **((2),** $R_2$ = H, $R_3$ = $CH_3$) and adipyl-7-ADCA **((2),** $R_2$ = adipyl, $R_3$ = $CH_3$) were adjusted using 4N NaOH and 6N $H_2SO_4$ to 2.7 (Table 5a), 4.2 (Table 5b) and 6.0 (Table 5c). At t=0 a sample was taken of the obtained solutions (before bisulfite was added). At 19±3°C, to each solution a solution of 25% sodium bisulfite was added with a flow of 30 ml/hr (60 $ml.l^{-1}.hr^{-1}$ Samples (2 ml) were taken at t=5, 20, 40 and 60 minutes. Samples for 6-APA analysis were diluted twice in phosphate buffer pH 7.5 and analyzed by LC-MS. Samples for 7-ADCA and adipyl-7-ADCA analysis were directly diluted 151x and measured by HPLC.

Table 5a: Degradation as a function of time at pH=2.7.

| Time (min) | 6-APA | | Adipyl-7-ADCA | | 7-ADCA | |
|---|---|---|---|---|---|---|
| | mg | % | g | % | g | % |
| 0 | 11.5 | 100 | 6.28 | 100 | 0.09 | 100 |
| 5 | 9.0 | 79 | 6.32 | 101 | 0.10 | 106 |
| 20 | 0.4 | 3 | 6.44 | 102 | 0.10 | 107 |
| 40 | 0.2 | 1 | 6.30 | 100 | 0.09 | 103 |
| 60 | 0.1 | 1 | 6.28 | 100 | 0.09 | 105 |

Table 5b: Degradation as a function of time at pH=4.2.

| Time (min) | 6-APA | | Adipyl-7-ADCA | | 7-ADCA | |
|---|---|---|---|---|---|---|
| | mg | % | g | % | g | % |
| 0 | 6.5 | 100 | 6.08 | 100 | 0.08 | 100 |
| 5 | 2.0 | 30 | 6.10 | 100 | 0.08 | 101 |
| 20 | 0.3 | 4 | 6.09 | 100 | 0.08 | 102 |
| 40 | 0.0 | 0 | 6.11 | 100 | 0.09 | 111 |

Table 5c: Degradation as a function of time at pH=6.0.

| Time (min) | 6-APA | | Adipyl-7-ADCA | | 7-ADCA | |
|---|---|---|---|---|---|---|
| | mg | % | g | % | g | % |
| 0 | 4.0 | 100 | 5.87 | 100 | 0.08 | 100 |
| 5 | 1.7 | 43 | 5.89 | 100 | 0.08 | 101 |
| 20 | 0.1 | 3 | 6.17 | 105 | 0.09 | 116 |
| 40 | 0.0 | 0 | 5.88 | 100 | 0.07 | 97 |
| 60 | 0.0 | 0 | 5.88 | 100 | 0.07 | 92 |

## Example 6

**Degradation of adipyl-6-APA vs. adipyl-7-ADCA by $NaHSO_3$ at 46±2°C**

Adipyl-7-ADCA **((2),** $R_2$ = adipyl, $R_3$ = $CH_3$; 95% purity, 7.06 mg, ~1 equiv.) and adipyl-6-APA **((1),** $R_1$ = adipyl; 44% purity, 14.65 mg, ~1 equiv.) were dissolved in 4 mL of phosphate buffer, pH=6-7. In order to obtain a clear solution, the sample was placed for 2-3 minutes in ultrasonic bath. 550μl was transferred to an NMR tube and placed into a spectrometer (700 MHz, preheated at 46±2°C). The sample was kept in the spectrometer and a spectrum was recorded at the times indicated in Table 6.

In a next experiment, adipyl-7-ADCA **((2),** $R_2$ = adipyl, $R_3$ = $CH_3$; 95% purity, 6.66 mg, ~1 equiv.), adipyl-6-APA ((**1**), $R_1$ = adipyl; 44% purity, 14.57 mg, ~1 equiv.) and $NaHSO_3$ (9.43 mg, 4.7 equiv.) were dissolved in 4 mL of phosphate buffer, pH=6-7. In order to obtain a clear solution, the sample was placed for 2-3 minutes in ultrasonic bath. 550μl was transferred to an NMR tube and placed into a spectrometer (700 MHz, preheated at 46±2°C). The sample was kept in the spectrometer and a spectrum was recorded at the times indicated in Table 6.

Decomposition of the title compounds was determined by measurement of the integral of the doublet at 5.4 ppm (β-lactam ring of adipyl-6-APA) and the doublet at 5.05 ppm (β-lactam ring of adipyl-7-ADCA). The initial increase of both adipyl-6-APA and adipyl-7-ADCA signals is probably a consequence of the measurement being taken before the sample has reached the desired temperature of 46±2°C. The results are depicted in Table 6 indicating that, in the presence of sulfite, adipyl-6-APA is degraded preferentially over adipyl-7-ADCA.

Table 6: Degradation of adipyl-6-APA vs. adipyl-7-ADCA by $NaHSO_3$ at 46±2°C and pH=6.

| Time (min) | Adipyl-6-APA (integral of doublet at 5.4 ppm) | | Adipyl-7-ADCA (integral of doublet at 5.05 ppm) | |
|---|---|---|---|---|
| | No $NaHSO_3$ | $NaHSO_3$ | No $NaHSO_3$ | $NaHSO_3$ |
| 2 | 5.89 | 5.40 | 5.27 | 4.64 |
| 30 | 6.22 | 5.80 | 5.65 | 5.24 |
| 45 | 6.23 | 5.78 | 5.67 | 5.26 |
| 60 | 6.19 | 5.87 | 5.67 | 5.35 |
| 75 | 6.13 | 5.70 | 5.61 | 5.33 |
| 150 | 5.98 | 5.34 | 5.55 | 5.20 |
| 265 | 5.70 | 5.03 | 5.46 | 5.25 |
| 390 | 5.68 | 4.51 | 5.64 | 5.12 |
| 540 | 5.49 | 4.09 | 5.63 | 5.08 |
| 790 | 5.44 | 3.38 | 5.79 | 5.14 |

## Claims

1. A process for the degradation of a compound of general formula (1) in a mixture comprising said compound of general formula (1) and a compound of general formula (2)

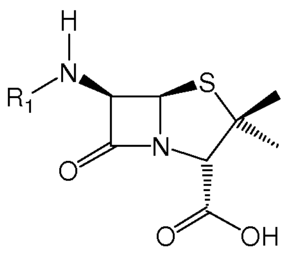

(**1**)

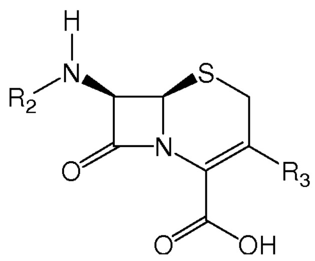

(**2**)

wherein $R_1$ and $R_2$ are independently chosen from the list consisting of adipyl and hydrogen and wherein $R_3$ is methyl, **characterized in that** said degradation is carried out in the presence of sulfite.

2. Process according to claim 1 wherein $R_1$ is the same as $R_2$.

3. Process according to claim 1 wherein $R_1$ is hydrogen and $R_2$ is adipyl.

4. Process according to any one of claims 1 to 3 wherein said sulfite is a bisulfite.

5. Process according to any one of claims 1 to 4 wherein said degradation is carried out at a pH between 3 and 9.

6. Use of sulfite for the degradation of a compound of formula (1) in an aqueous mixture comprising said compound of general formula (1) and a compound of general formula **(2)**

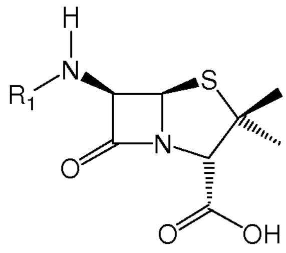

(1)

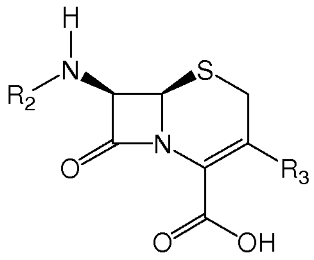

(2)

wherein $R_1$ and $R_2$ are independently chosen from the list consisting of adipyl and hydrogen and wherein $R_3$ is methyl.

7. Use according to claim 6 wherein said aqueous mixture is a waste stream.

**Patentansprüche**

1. Verfahren zum Abbau einer Verbindung der allgemeinen Formel (1) in einem Gemisch, das die Verbindung der allgemeinen Formel (1) und eine Verbindung der allgemeinen Formel (2) umfasst,

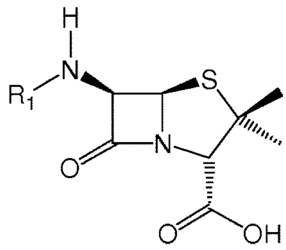

(1)

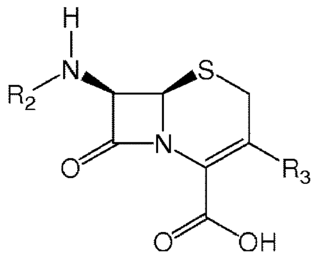

(2)

wobei $R_1$ und $R_2$ unabhängig voneinander aus der Liste bestehend aus Adipyl und Wasserstoff ausgewählt sind und wobei $R_3$ Methyl ist, **dadurch gekennzeichnet, dass** der Abbau in Gegenwart von Sulfit durchgeführt wird.

2. Verfahren nach Anspruch 1, bei dem $R_1$ mit $R_2$ identisch ist.

3. Verfahren nach Anspruch 1, bei dem $R_1$ Wasserstoff ist und $R_2$ Adipyl ist.

4. Verfahren nach einem der Ansprüche 1 bis 3, bei dem es sich bei dem Sulfit um ein Bisulfit handelt.

5. Verfahren nach einem der Ansprüche 1 bis 4, bei dem der Abbau bei einem pH-Wert zwischen 3 und 9 durchgeführt wird.

6. Verwendung von Sulfit zum Abbau einer Verbindung der allgemeinen Formel (1) in einem wässrigen Gemisch, das die Verbindung der allgemeinen Formel (1) und eine Verbindung der allgemeinen Formel (2) umfasst,

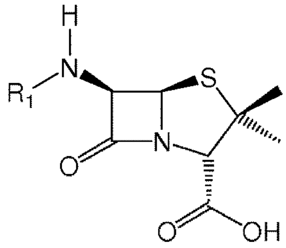

(1)

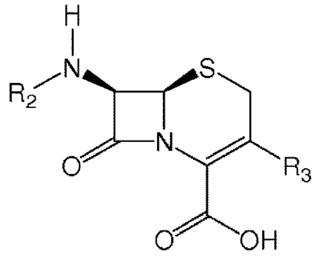

(2)

wobei $R_1$ und $R_2$ unabhängig voneinander aus der Liste bestehend aus Adipyl und Wasserstoff ausgewählt sind und wobei $R_3$ Methyl ist.

**7.** Verwendung nach Anspruch 6, bei dem es sich bei dem wässrigen Gemisch um einen Abfallstrom handelt.

## Revendications

**1.** Procédé de dégradation d'un composé de formule générale (1) dans un mélange comprenant ledit composé de formule générale (1) et un composé de formule générale (2)

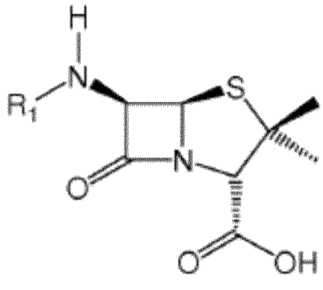

(1)

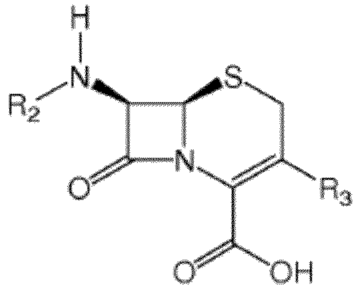

(2)

où $R_1$ et $R_2$ sont indépendamment choisis dans la liste constituée d'adipyle et d'hydrogène et où $R_3$ est méthyle, **caractérisé en ce que** ladite dégradation est conduite en présence de sulfite.

**2.** Procédé selon la revendication 1, dans lequel $R_1$ est identique à $R_2$.

**3.** Procédé selon la revendication 1, dans lequel $R_1$ est hydrogène et $R_2$ est adipyle.

**4.** Procédé selon l'une quelconque des revendications 1 à 3 dans lequel ledit sulfite est un bisulfite.

**5.** Procédé selon l'une quelconque des revendications 1 à 4 dans lequel ladite dégradation est conduite à un pH compris entre 3 et 9.

**6.** Utilisation de sulfite pour la dégradation d'un composé de formule (1) dans un mélange aqueux comprenant ledit composé de formule générale (1) et un composé de formule générale (2)

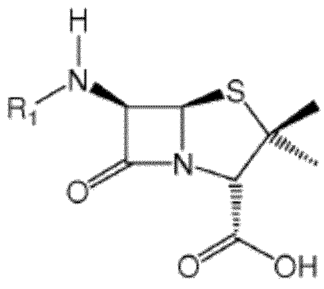

(1)

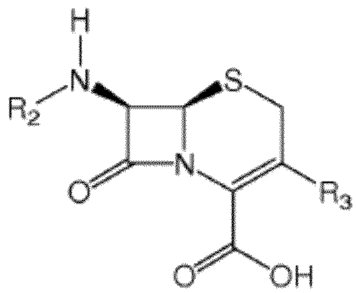

(2)

où $R_1$ et $R_2$ sont indépendamment choisis dans la liste constituée d'adipyle et d'hydrogène et où $R_3$ est méthyle.

**7.** Utilisation selon la revendication 6 dans laquelle ledit mélange aqueux est un flux de déchets.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 9305158 A
- WO 9504148 A
- WO 9504149 A
- WO 9848036 A
- WO 9848034 A
- WO 9848035 A
- GB 1259204 A

### Non-patent literature cited in the description

- **FLOREY et al.** Antibiotics. Oxford University Press, 1949, vol. II, 802
- **CLARKE et al.** The Chemistry of Penicillin. Princeton University Press, 1949, 207-242